# EUROPÄISCHE PATENTSCHRIFT

(11) **EP 1 401 531 B1**
(45) Veröffentlichungstag und Bekanntmachung des Hinweises auf die Patenterteilung: **17.08.2005**
(21) Anmeldenummer: 02748755.2
(22) Anmeldetag: 10.06.2002
(51) Int. Cl.: A61N 1/20

(54) **NADELELEKTRODE**
NEEDLE ELECTRODE
ELECTRODE AIGUILLE

(30) Priorität: 21.06.2001 DE 10129912
(43) Veröffentlichungstag der Anmeldung: 31.03.2004
(73) Patentinhaber: EFMT Entwicklungs- und Forschungszentrum Für Mikrotherapie GmbH, 44799 Bochum (DE)
(72) Erfinder: GRÖNEMEYER, Dietrich, H., W., 45549 Sprockhövel (DE); SAHINBAS, Hüseyin, c/o GIMT, 44799 Bochum (DE); BRACKE, Andreas, 44795 Bochum (DE); DELI, Martin, 45472 Mülheim (DE); DENK, Marion, 44803 Bochum (DE); GONSCHOREK, Katja, 44789 Bochum (DE); RICHTER, Jörn, 48151 Münster (DE); SPEDER, Jürgen, 44807 Bochum (DE)
(74) Vertreter: Thiel, Christian
(86) Internationale Anmeldenummer: PCT/EP2002/006345
(87) Internationale Veröffentlichungsnummer: WO 2003/000339

(56) Entgegenhaltungen:
- EP-A- 0 378 132
- DE-A- 4 344 986
- US-A- 5 873 849

## Beschreibung

Die Erfindung betrifft eine Nadelelektrode für die Therapie, insbesondere für die perkutane Galvanotherapie bei Tumoren, die für die Darstellung in bildgebenden Verfahren geeignet ist. Ferner betrifft die Erfindung ein Verfahren zur Herstellung der erfindungsgemäßen Nadelelektrode.

Zur Behandlung von Primärtumoren, Hauttumoren oder Metastasen werden heutzutage verschiedene Therapien zur Verfügung gestellt. Zu nennen wäre hier die operative Entfernung des Tumors, die Kryotherapie, die Hyperthermie, die Chemotherapie, die Alkoholablation, die Radiofrequenzablation oder die elektrochemische Therapie.

Unter der elektrochemischen Tumortherapie (ECT) wird die Galvanotherapie verstanden. Diese Methode wird vor allem bei Tumoren angewandt, die aus funktionalen oder ästhetischen Gründen inoperabel sind, nicht mehr bestrahlt werden können oder Resistenzen gegen eine Chemotherapie gebildet haben. Bei der elektrochemischen Tumortherapie (ECT) oder Galvanotherapie fließt durch das Anlegen von Elektroden an tumoröses Gewebe, wie z. B. Hautmetastasen, Lymphknotenmetastasen oder isolierte Organmetastasen, ein Gleichstrom durch das tumoröse Gewebe. Bei einer ausreichenden Gesamtstrommenge kommt es zur Abtötung des Tumorgewebes und im Extremfall zur Nekrotisierung (völliges Absterben).

Sobald eine Gleichspannung an den Elektroden anliegt, ändern sich durch verschiedene chemoelektrische Vorgänge der pH-Wert und die elektrische Ladung des Tumorgewebes. Das dadurch aufgebaute Stromfeld im Bereich des Tumors bewirkt eine Wanderung von geladenen Teilchen im Stromfeld.

Negativ geladene Teilchen (Anionen) wandern zum elektrisch positiv geladenen Pol (Anode), während positiv geladene Teilchen (Kationen) zur Kathode (negativer Pol) wandern. Diese sogenannte Ladungstrennung wird auch Dissoziation genannt. Bei der Dissoziation werden auch größere geladene Teilchen wie Proteine je nach ihrer Ladung getrennt. Besonders wichtig für die Zerstörung der Tumorzellen ist die polarisierende Veränderung der Zellmembranen, so daß die Stoffwechselfunktionen der Zellmembranen (Elektrolytpumpen, Nährstoffpumpen, usw.) empfindlich gestört werden. Das spezifische Gleichgewicht der Krebszellen für wichtige Lebensprozesse wird somit unterbrochen, was zum Absterben der Zelle führt.

Dieses Heilverfahren findet zunehmend in der Onkologie Anwendung, da Tumorgewebe einen wesentlich geringeren elektrischen Widerstand hat als gesundes Gewebe. Der Stromfluß konzentriert sich vorwiegend auf das bösartige Gewebe und ermöglicht somit eine gezielte Abtötung von malignem (bösartigem) Gewebe. Das abgetötete Tumorgewebe wird auf natürlichem Wege z. B. durch erhöhte Freßzellenaktivität abgebaut, eliminiert und durch Narbengewebe ersetzt.

Eine erweiterte Form dieser elektrochemischen Therapie ist die Kombination mit Chemotherapien. Die zerstörerische Wirkung des Gleichstroms auf das Tumorgewebe kann erhöht werden, indem Zytostatika (Chemotherapiesubstanzen), wie z. B. Mitomycin, Adriblatin, Epirubicin und Cisplatin, gleichzeitig in den Tumor gebracht werden. Bei diesen Zytostatika handelt es sich meist um kationische Substanzen, die über die Anode im elektrischen Feld durch das Tumorgewebe zur Kathode wandern. Auf diese Art und Weise werden Zytostatika gezielt und konzentriert in das Tumorgewebe eingebracht und verteilt, wo ihre Wirkung sich optimal entfalten können. Bei der systemischen Chemotherapie oder der lokalen Zytostatikaperfusion ohne Elektrotherapie ist das Einbringen der Substanzen dagegen nicht immer steuerbar, so daß gesundes Gewebe auch zerstört werden kann.

Ein weiterer Effekt ist die Veränderung der Zellmembranpotentiale durch das Stromfeld. Dadurch öffnen sich die Zellen, so daß Zytostatika stärker als sonst aufgenommen werden. Aufgrund des durch das Stromfeld bedingten sauren Milieus der Anode kommt es zu einer höheren Aktivität der Zystostatika. Der Wirkungskoeffizient wird somit um ein Vielfaches erhöht.

Für die elektrotherapeutische Behandlung werden daher Elektroden, die als dünne Nadel und für die kombinierte Behandlung mit der Chemotherapie als Kanüle ausgebildet sind, verwendet. Herkömmliche Nadeln bzw. Kanülen bestehen aus Kupfer oder Edelstahl, der auch mit Kupfer legiert sein kann. Ein großer Nachteil dieser Nadeln bzw. Kanülen ist die elektrochemische Zersetzung der Kupferlegierung (galvanische Korrosion). Bei Gegenwart von Kupfer sind die entstehenden Kupferionen in hohen Konzentrationen für den Organismus giftig. Darüber hinaus werden die Leitfähigkeit und Beständigkeit der Nadel/Kanüle verringert. Das Stromfeld wird dadurch nicht optimal aufgebaut, was eine Verschlechterung der Behandlungsbedingung bedeutet. Negative Wechselwirkungen zwischen den eingebrachten Zystostatika und den Kupferionen im Hinblick auf das Tumorgewebe sowie auf das gesunde Gewebe können nicht völlig ausgeschlossen werden.

Bei der Wahl des Materials für Nadelelektroden und Kanülen müssen daher einerseits die physikalischen Eigenschaften (Leitfähigkeit, Beständigkeit, Festigkeit) und andererseits hinsichtlich der Abstoßungs-, Entzündungsgefahr des Gewebes die Verträglichkeit berücksichtigt werden.

Angesichts dieser Anforderungen besteht die Aufgabe der Erfindung darin, eine Nadelelektrode bereitzustellen, welche eine sehr gute Leitfähigkeit des elektrischen Stromes, eine große Beständigkeit während des Stromfeldaufbaus, eine gute Verträglichkeit (Bio-kompatibilität) aufweist und darüber hinaus inert gegenüber Zystostatika ist. Weiterhin sollte eine solche Nadelelektrode an der jeweiligen Anwendungsstelle keine oberflächlichen Verbrennungen und/oder Narben größeren Ausmaßes hinterlassen. Es wäre auch wünschenswert, über ein Verfahren zur Herstellung solcher Nadelelektroden zu verfügen, das die obengenannten Eigenschaften mit sich bringt.

EP-A-0 378 0132 beschreibt eine Nadelelektrode für die iontophoretische Medikation, bei der ein Elektrodenkörper zunächst mit einer Kunststoffschicht isoliert und dann mit einer umgebenden Platinschicht bedeckt ist.

DE 43 44 986 A beschreibt ein Verfahren und eine Vorrichtung zur Durchführung einer medizinischen Elektrotherapie unter gleichzeitiger Verabreichung von Therapeutika unter Verwendung nicht-metallischer Elektroden. In der Beschreibung wird die Verwendung von u.a. Titan als Elektrodenmaterial angesprochen. Wegen der Löslichkeit metallischer Elektrodenmaterialien unter elektrotherapeutischen Bedingungen wird aber die Verwendung natürlicher, nicht-metallischer Elektrodenmaterialien empfohlen.

Zur Lösung dieser Aufgabe schlägt die Erfindung, ausgehend von einer Nadelelektrode der eingangs genannten Art vor, daß diese einen mit Platin beschichteten Titankörper aufweist. Insbesondere handelt es sich um eine Nadelelektrode mit platinbeschichtetem Titankörper.

In der Medizin wird Titan bei der Herstellung von Knochennägeln, Prothesen, Nadeln usw. aufgrund seiner bio-kompatiblen Eigenschaft auf den menschlichen Organismus und seiner besonderen Widerstandsfähigkeit gegen Stoß und Schlag eingesetzt. Auch die physikalischen Eigenschaften des Titans, nämlich die sehr gute Leitfähigkeit für elektrischen Strom, stellt somit ein ideales Material für die Verwendung als Nadelelektrode bzw. Kanüle dar. Aufgrund seiner Korrosions- und Lochfraßgefahr wird Titan oder seine Legierungen jedoch bisher kaum als Elektrode verwendet.

Zur Verbesserung seiner Korrosionsbeständigkeit kann eine passivierende oxidierende Deckschicht auf dem jeweiligen Körper/Gegenstand aus Titan aufgebracht werden. Für die Elektrotherapie stellt dies keine zufriedenstellende Lösung dar.

Aus diesem Grund wird erfindungsgemäß Platin auf dem Titankörper aufgebracht. Platin gehört zu den Edelmetallen, die wenig elektrochemische Korrosion aufweisen. Platinelektroden sind bekanntermaßen gute Elektroden, da sie sowohl eine gute elektrische Leitfähigkeit als auch eine gute Beständigkeit besitzen. Durch die Beschichtung des Titankörpers mit Platin wird die Korrosions- und Lochfraßbeständigkeit der Nadelelektrode bei gleichbleibender hoher elektrischer Leitfähigkeit erhöht. Die Herstellung einer Nadelelektrode aus 100 %igem Platin wäre aufgrund des hohen Preises von Platin aus finanzieller Sicht und hinsichtlich der daraus resultierenden hohen Behandlungskosten nicht besonders sinnvoll. Des weiteren kommen Platin bzw.

Platinlegierungen auch deshalb für Elektroden nicht in Frage, da Platin besonders weich ist. Für das Einbringen der Nadelelektrode in den menschlichen Körper ist die Festigkeit eine wesentliche Voraussetzung.

Untersuchungen haben gezeigt, daß die Beschichtung eines Titankörpers mit Edelmetallen sich als äußerst schwierig gestaltet. Edelmetallschichten bleiben selten dauerhaft auf dem Titankörper haften. Sie lösen sich innerhalb kürzester Zeit ab bzw. auf. Für die Elektrotherapie muß der Titankörper aber dauerhaft oder zumindest für die Dauer der Behandlung sicher mit der Platinschicht verbunden sein. Dies wird mit der PVD-Beschichtung erreicht.

Zweckmäßig ist deshalb eine Platinbeschichtung, die mit Hilfe des PVD-Verfahrens (Physical Vapour Deposition) gebildet wird. Man unterscheidet im wesentlichen drei verschiedene Verfahrenstechniken. Bei einer bevorzugten Ausführung wird Platin in einer Vakuumkammer verdampft, ionisiert und beschleunigt und anschließend auf dem Titankörper niedergeschlagen. Durch die hohe Beschleunigung der beaufschlagten Ionen bleibt die Platinschicht als dünne Schicht relativ dauerhaft auf dem Titankörper haften.

Andere Techniken wie das Zerstäubung-Edelgasplasma-Technik, das lonenstrahl-Abtragung-Technik oder Kombinationen dieser Techniken, wie das Plasma-unterstützte Aufdampfen oder das Ionen-Implantieren, kommen ebenso gut für die Platinbeschichtung in Frage. [Lit.: Römpp, Chemie Lexikon, Thieme Verlag, 9., erweiterte und neubearbeitete Auflage]

Zur Absicherung der Korrosionsbeständigkeit und der Haftungsdauer der Platinschicht der Nadelelektrode beträgt die Dicke der Platinbeschichtung zwischen 0,1 µm bis 3,0 µm, vorzugsweise etwa 1,0 µm. Der Durchmesser des Titankörpers liegt zwischen 0,1 mm und 1,0 mm, vorzugsweise bei 0,5 bis 0,8 mm. Überraschend wurde festgestellt, daß die Korrosionsbeständigkeit der Nadelelektrode abhängig ist von dem Verhältnis zwischen dem Durchmesser des Titankörpers und der Dicke der Platinschicht. Das Verhältnis liegt zwischen 1 : 0,00075 und 1 : 0,0025 (∅ Titankörper : Pt-Schicht). Dünne Titankörper werden zweckmäßigerweise mit einer relativ dickeren Beschichtung versehen, um die Korrosionsbeständigkeit zu garantieren.

Als besonders zweckmäßig hat sich das Verhältnis 1 : 0,00125 erwiesen.

Die erfindungsgemäße Nadelelektrode ist für die Darstellung in bildgebenden Systemen geeignet, insbesondere für die Kernspin(resonanz)tomographie, die Computertomographie und für das Ultraschall-Verfahren. Während der Behandlung ist eine optische Darstellung des Tumors und der Nadelelektroden unerläßlich. Die Nadelelektroden werden durch die Haut und Körpergewebe in den Tumor eingeführt. Die Grenze zwischen tumorösem und gesundem Gewebe muß exakt zu erkennen sein, damit nicht gesunde Zellen zerstört werden, und die Position der Nadel muß exakt zu bestimmen sein.

Bevorzugt weist die Nadelelektrode eine Länge von 3 bis 20 cm, vorzugsweise 6 bis 14 cm auf. Somit können sowohl oberflächliche Hautmetastasen als auch Weichteiltumoren behandelt werden.

Eine weitere bevorzugte Ausführung der Erfindung ist eine mit einem nicht-leitenden, isolierenden Polymer überzogene Nadelelektrode aus Titan mit einer Platinbeschichtung. Insbesondere bei der Behandlung von tiefliegenden Tumoren, also keine Hautmetastasen, werden die Nadelelektroden perkutan bis zum Tumor eingeführt. Die perkutane Einstichlänge hängt von dem Ort des Tumors ab. An der Einstichlänge sind normalerweise gesunde Zellen zugegen. Durch das Anlegen einer Spannung werden in diesem Bereich gesunde Zellen irritiert.

Mit der isolierten Nadelelektrode wird gesundes Gewebe an der Einstichlänge nicht verletzt. Die elektrische Spannung entsteht ausschließlich am Tumor, so daß nur dort das Stromfeld mit seiner zerstörerischen Wirkung aufgebaut wird. Aus diesem Grund wird die isolierende Schicht derart ausgebildet, daß die Spitze bzw. ein bestimmter Abstand am Ende der Nadelelektrode nicht beschichtet wird. Der Abstand richtet sich danach, wie weit die Nadelelektrode in den Tumor hineinragt. Dies ist wiederum abhängig von der Größe des Tumors. Dieser Abstand bis zur Nadelspitze wird hier allgemein als Spitzenbereich definiert. Soweit die Elektrode isolierend beschichtet ist, kann sich die vorhandene Platinbeschichtung auf den Spitzenbereich beschränken, wobei eine gewisse Überschneidung der Beschichtungen wünschenswert ist.

Herkömmliche Edelstahlnadel/Kanüle weisen keine isolierende Schicht auf; sie hinterlassen häufig Verbrennungsmale an der Einstichstelle.

Aus diesem Grund kann die erfindungsgemäße Nadelelektrode auch für andere medizinische Instrumente verwendet werden, die für elektrolytische oder elektrochemische Behandlung, insbesondere zur elektrolytischen Ablösung von Occlusionswendeln für endovaskuläre oder endovasale Technik wie z. B. vaskulärer Aneurysmen, eingesetzt werden.

Die Dicke der isolierenden Beschichtung hängt von den jeweils verwandten Materialien und Verfahren ab und muß ausreichen, um eine gute Haftung zu ergeben und die Elektrode im beschichteten Bereich zuverlässig zu isolieren.

Als Polymer wird Parylen N, bevorzugt Parylen D und besonders bevorzugt Parylen C verwandt. Sie besitzen hervorragende dielektrische Eigenschaften und sind ausgezeichnete Barrierekunststoffe. Das Monomer wird mittels CVD-Polymerisation (Chemical vapour deposition) auf der Nadel polymerisiert und abgeschieden. Dem CVD-Verfahren liegt der Gorham Prozeß zugrunde.

Eine andere bevorzugte Ausführung der Erfindung ist die isolierende Beschichtung mit PTFE (Polytetrafluorethylen). Die Beschichtung wird hierbei vorzugsweise über ein Sprayverfahren aufgebracht.

Die Schichtdicke des Polymers liegt zwischen 0,01 mm bis 0,09 mm, vorzugsweise zwischen 0,025 mm bis 0,05 mm.

Vorteile dieser isolierenden Beschichtung ist die Verminderung der Reibung im trockenen Zustand, die elektrische Isolierung und die sehr dünne, transparente Schicht.

Bevorzugt ist die Nadelelektrode als Kanüle für die Elektro-Chemo-Therapie ausgebildet. Unter Elektro-Chemo-Therapie wird die Kombinationsbehandlung von Galvanotherapie und Chemotherapie verstanden.

Darüber hinaus betrifft die Erfindung ein Verfahren zur Herstellung der erfindungsgemäßen Nadelelektroden für die Elektrotherapie, insbesondere für die perkutane Galvanotherapie bei Tumoren, wobei der Titankörper der Nadelelektrode mit Platin mittels eines PVD-Verfahrens beschichtet wird.

Bei dem PVD-Verfahren handelt es sich insbesondere um folgende Verfahrensschritte
- Verdampfen und lonisieren von Platinmetall in einer Vakuumkammer,
- ggf. Zugabe von Reaktivgasen,
- Anlegen einer elektrischen Spannung,
- Beschleunigung der gebildeten Ionen auf den Titankörper und Niederschlagung desselben darauf.

Mit diesem Verfahren wird eine ideale Beschichtung des Titankörpers mit Platin ermöglicht. Durch die Zugabe von Reaktivgasen bildet sich der eigentliche Schichtstoff, der sich auf dem in einiger Entfernung befindlichen Titankörper abscheidet.

Zweckmäßig wird mit Hilfe des Ausscheidungsprozesses nach Gorham ein nicht-leitendes Polymer auf die Nadel aufgebracht. Zur Beschichtung werden die Parylen-Polymere aus der Gasphase abgeschieden (Gorham Prozess). Zuerst wird das feste Dimer Di-para-Xylylen bei etwa 150 °C verdampft. Bei etwa 680 °C wird das Dimer quantitativ an den beiden Methylen-Methylen-Bindungen aufgespalten. Hierbei entsteht stabiles monomeres p-Xylylen. Das Monomer polymerisiert dann bei Raumtemperatur in der Beschichtungskammer auf dem Titankörper.

Eine andere bevorzugte Ausführung der Erfindung ist die isolierende Beschichtung mit PTFE (Polytetrafluoretylen). Die Beschichtung wird hierbei vorzugsweise über ein Sprayverfahren aufgebracht.

Nachstehend wird die Erfindung anhand von Untersuchungen näher erläutert.

### Beispiel 1

### Versuche zur Korrosions- und Lochfrasbeständigkeit der Nadelelektroden mit verschiedenen Beschichtungen

Zur Ermittlung der Korrosions- und Lochfraßbeständigkeit der verschiedenen Gold- und Platinbeschichtungen auf den ECT-Titan-Nadeln werden je zwei Nadeln im gleichen Abstand in eine Schweineleber eingeführt und mit Gleichstrom beaufschlagt. Die Versuchsdauer wird gestaffelt durchgeführt.

| | Beschichtung | Schichtdicke [µm] | Elektroden-Abstand [mm] | Gleichstrom [mA] | Zeit [min:s] | Durchmesser Titankörpers |
|---|---|---|---|---|---|---|
| 1. | Au | / | 25 | 80 | 10 | ⌀ 0,8 mm |
| 2. | Pt | 1 µm | 25 | 80 | 10 | ⌀ 0,8 mm |
| 3. | Pt | 1 µm | 25 | 80 | 20 | ⌀ 0,5 mm |
| 4. | Aurobond getempert + Pt | / | 25 | 80 | 20 | ⌀ 0,8 mm |
| 5. | Flash Gold | 0,2 µm | 25 | 80 | 20 | ⌀ 0,5 mm |

### Ergebnis:

Der Versuch zeigt, daß die Platinbeschichtung (Nr. 2 und 3) im Gegensatz zu den Goldbeschichtungen kaum durch Korrosion und Lochfras beeinträchtigt werden. Die Goldbeschichtungen zeigen bereits nach kurzer Zeit eine Veränderung in der Oberflächenstruktur.

Bei der Platinbeschichtung mit kleinerem Titankörper-Durchmesser (⌀ 0,5 mm) wird eine geringe Auflösung nach 20 min festgestellt. Dieser kann durch Erhöhung der Schichtdicke entgegen gewirkt werden.

## Patentansprüche

1. Nadelelektrode für die Therapie, insbesondere für die perkutane Galvanotherapie bei Tumoren, die in bildgebenden Verfahren darstellbar ist, **dadurch gekennzeichnet, daß** sie einen platinbeschichteten Titankörper aufweist.

2. Nadelelektrode nach Anspruch 1, **dadurch gekennzeichnet, daß** die Platinbeschichtung eine PVD-Beschichtung ist.

3. Nadelelektrode nach einem der Ansprüchen 1 oder 2, **dadurch gekennzeichnet, daß** die Dicke der Platinbeschichtung zwischen 0,1 µm bis 3,0 µm, vorzugsweise etwa 1,0 µm, beträgt.

4. Nadelelektrode nach einem der Ansprüche 1 bis 3, **dadurch gekennzeichnet, daß** der Durchmesser des Titankörpers zwischen 0,1 mm und 1,0 mm, vorzugsweise zwischen 0,5 mm und 0,8 mm, liegt.

5. Nadelelektrode nach einem der vorstehenden Ansprüche, **dadurch gekennzeichnet, daß** sie außerhalb des Spitzenbereich mit einem elektrisch nicht-leitenden, isolierenden Polymer überzogen ist.

6. Nadelelektrode nach Anspruch 5, **dadurch gekennzeichnet, daß** als Polymer Parylen N, Parylen D oder besonders bevorzugt Parylen C verwandt wird.

7. Nadelelektrode nach Anspruch 5, **dadurch gekennzeichnet, daß** die isolierende Beschichtung aus Polytetrafluorethylen (PTFE) besteht.

8. Nadelelektrode nach einem der Ansprüche 5 bis 7, **dadurch gekennzeichnet, daß** die Schichtdicke des Polymers zwischen 0,001 mm bis 0,09 mm liegt, vorzugsweise zwischen 0,0025 mm bis 0,05 mm.

9. Nadelelektrode nach einem der vorstehenden Ansprüche, **dadurch gekennzeichnet, daß** sie als Kanüle für die Elektro-Chemo-Therapie ausgebildet ist.

10. Nadelelektrode nach Anspruch 1, **dadurch gekennzeichnet, daß** sie eine Länge von 3 bis 20 cm, vorzugsweise 6 bis 14 cm, aufweist.

11. Verfahren zur Herstellung einer Nadelelektrode für die Elektrotherapie, insbesondere für die perkutane Galvanotherapie bei Tumoren, **dadurch gekennzeichnet, daß** eine Nadelelektrode aus Titan mittels eines PVD-Verfahrens mit Platin beschichtet wird.

12. Verfahren nach Anspruch 11, **dadurch gekennzeichnet, daß** das PVD-Verfahren die Verfahrensschritte
- Verdampfen und lonisieren von Platinmetall in einer Vakuumkammer,
- ggf. Zugabe von Reaktivgasen,
- Anlegen einer elektrischen Spannung,
- Beschleunigung der gebildeten Ionen auf den Titankörper und Niederschlagung desselben darauf
aufweist.

13. Verfahren nach Anspruch 11 oder 12, **dadurch gekennzeichnet, daß** die Elektrode außerhalb des Spitzenbereichs weiterhin mit einem nicht-leitenden Polymer überzogen wird.

14. Verfahren nach Anspruch 13, **dadurch gekennzeichnet, daß** als Polymer Parylen N, bevorzugt Parylen D, besonders bevorzugt Parylen C, verwandt wird.

15. Verfahren nach einem der Ansprüche 13 oder 14, **dadurch gekennzeichnet, daß** das nicht-leitende Polymer mittels des Ausscheidungsprozesses nach Gorham angewandt wird.

16. Verfahren nach Anspruch 13, **dadurch gekennzeichnet, daß** die isolierende Beschichtung mittels eines Sprayverfahrens erzeugt wird.

17. Verfahren nach Anspruch 16, **dadurch gekennzeichnet, daß** zur Beschichtung Polytetrafluorethylen (PFTE) verwandt wird.

## Claims

1. Needle electrode for therapy, especially percutaneous galvanotherapy of tumors, which can be visualized by image-generating procedures, **characterized in that** it has a platinum-coated titanium body.

2. Needle electrode according to claim 1, **characterized in that** the platinum coating is a PVD coating.

3. Needle electrode according to any of claims 1 or 2, **characterized in that** the thickness of the platinum coating is between 0.1 micron and 3.0 microns, the preferred thickness being approx. 1.0 micron.

4. Needle electrode according to any of claims 1 to 3, **characterized in that** the diameter of the titanium body is between 0.1 mm and 1.0 mm, preferably between 0.5 mm and 0.8 mm.

5. Needle electrode according to any of the above claims, **characterized in that** the needle electrode, with the exception of the needle tip area, is covered with an electrically non-conductive, insulating polymer.

6. Needle electrode according to claim 5, **characterized in that** the polymer used is parylene N, parylene D or preferably parylene C.

7. Needle electrode according to claim 5, **characterized in that** the insulating coating consists of polytetrafluorethylene (PTFE).

8. Needle electrode according to any of claims 5 to 7, **characterized in that** the layer thickness of the polymer is between 0.001 mm and 0.09 mm, preferably between 0.0025 mm and 0.05 mm.

9. Needle electrode according to any of the above claims, **characterized in that** it is designed as a cannula for electro-chemo-therapy.

10. Needle electrode according to claim 1, **characterized in that** it measures 3 to 20 cm in length, preferably 6 to 14 cm.

11. Process for the manufacture of a needle electrode for electrotherapy, especially for percutaneous galvanotherapy of tumors, **characterized in that** the titanium needle electrode is coated with platinum using a PVD process.

12. Process according to claim 11, **characterized in that** the PVD process comprises the following process stages:
- Platinum metal vaporization and ionization in a vacuum chamber,
- Addition of reactive gases - optional,
- Application of electric current,
- Acceleration of the ions formed onto the titanium body and deposition of the same on said body.

13. Process according to claims 11 or 12, **characterized in that** the electrode, except for the area of the electrode tip, is additionally coated with a non-conductive polymer.

14. Process according to claim 13, **characterized in that** the polymer used is parylene N, preferably parylene D, and more preferably parylene C.

15. Process according to any of claims 13 or 14, **characterized in that** the non-conductive polymer is applied using the Gorham deposition process.

16. Process according to claim 13, **characterized in that** the insulating coating is applied in a spray operation.

17. Process according to claim 16, **characterized in that** the material used for the coating is polytetrafluorethylene (PFTE).

## Revendications

1. Électrode aiguille pour le traitement, en particulier pour la galvanothérapie percutanée de tumeurs, qui est représentable dans des procédés d'imagerie, **caractérisée en ce qu'**elle présente un corps en titane revêtu de platine.

2. Électrode aiguille selon la revendication 1, **caractérisée en ce que** le revêtement de platine est un revêtement PVD.

3. Électrode aiguille selon l'une des revendications 1 ou 2, **caractérisée en ce que** l'épaisseur du revêtement de platine vaut entre 0,1 µm et 3,0 µm, de préférence environ 1, 0 µm.

4. Électrode aiguille selon l'une des revendications 1 à 3, **caractérisée en ce que** le diamètre du corps en titane est compris entre 0,1 mm et 1,0 mm, de préférence entre 0,5 mm et 0,8 mm.

5. Électrode aiguille selon l'une des revendications précédentes, **caractérisée en ce qu'**en dehors de sa zone de pointe, elle est revêtue d'un polymère isolant non conducteur électrique.

6. Électrode aiguille selon la revendication 5, **caractérisée en ce que** l'on utilise comme polymère du parylène N, du parylène D ou, de manière particulièrement préférée, du parylène C.

7. Électrode aiguille selon la revendication 5, **caractérisée en ce que** le revêtement isolant est composé de polytétrafluoroéthylène (PTFE).

8. Électrode aiguille selon l'une des revendications 5 à 7, **caractérisée en ce que** l'épaisseur de couche du polymère est comprise entre 0,001 mm et 0,09 mm, de préférence entre 0,0025 mm et 0,05 mm.

9. Électrode aiguille selon l'une des revendications précédentes, **caractérisée en ce qu'**elle est conformée en canule pour l'électro-chimiothérapie.

10. Électrode aiguille selon la revendication 1, **caractérisée en ce qu'**elle présente une longueur de 3 à 20 cm, de préférence de 6 à 14 cm.

11. Procédé de fabrication d'une électrode aiguille pour l'électrothérapie, en particulier pour la galvanothérapie de tumeurs, **caractérisé en ce qu'**une électrode aiguille en titane est revêtue de platine par un procédé PVD.

12. Procédé selon la revendication 11, **caractérisé en ce que** le procédé PVD comprend les étapes de :
- évaporation et ionisation de platine métal dans une chambre à vide ;
- éventuellement ajout de gaz réactifs ;
- application d'une tension électrique ;
- accélération des ions formés en direction du corps en titane et leur dépôt sur celui-ci.

13. Procédé selon la revendication 11 ou 12, **caractérisé en ce qu'**en dehors de la zone de pointe, l'électrode est revêtue de plus d'un polymère non conducteur.

14. Procédé selon la revendication 13, **caractérisé en ce que** l'on utilise comme polymère du parylène N, de préférence à du parylène D ou, de manière particulièrement préférée, du parylène C.

15. Procédé selon l'une des revendications 13 ou 14, **caractérisé en ce que** le polymère non conducteur est utilisé par le procédé de séparation de Gorham.

16. Procédé selon la revendication 13, **caractérisé en ce que** le revêtement isolant est produit par un procédé de pulvérisation.

17. Procédé selon la revendication 16, **caractérisé en ce que**, pour le revêtement, on utilise du polytétrafluoroéthylène (PTFE).
